# EUROPÄISCHE PATENTSCHRIFT

(11) **EP 1 913 928 B1**
(45) Veröffentlichungstag und Bekanntmachung des Hinweises auf die Patenterteilung: **09.12.2009**
(21) Anmeldenummer: 06021996.1
(22) Anmeldetag: 20.10.2006
(51) Int. Cl.: A61K 8/04, A61K 8/81, A61Q 1/10

(54) **Kosmetische Zubereitung zum Färben der Augenlider und der Augenbrauen**
Cosmetic composition for colouring eyelids and eyebrows
Composition cosmétique pour colorer les paupières et les sourcils

(43) Veröffentlichungstag der Anmeldung: 23.04.2008
(73) Patentinhaber: Faber-Castell AG, 90546 Stein (DE)
(72) Erfinder: Lugert, Gerhard, Dr., 90431 Nürnberg (DE); Appel, Tatiana, 90522 Oberasbach (DE)
(74) Vertreter: Mörtel & Höfner

(56) Entgegenhaltungen:
- EP-A- 1 247 514
- DE-U1-202004 018 813
- GB-A- 1 193 829

## Beschreibung

Die Erfindung betrifft eine kosmetische Zubereitung zum Färben der Augenlider und der Augenbrauen. Die Färbung der genannten Hautpartien erfolgte bisher hauptsächlich mit Hilfe relativ hoch viskoser Zubereitungen, die mit Hilfe von Bürsten, Pinseln u. dgl. aufgetragen wurden. Eine exakte Applikation ist auf die genannte Art nur schwer möglich. Außerdem ist ein mehrmaliges Aufnehmen von Zubereitung aus einem Vorratsbehälter erforderlich, um die gewünschte Hautpartie mit einem gleichmäßigen Farbauftrag zu versehen. Dabei ist es oft nicht zu vermeiden, dass Zubereitung von den Auftragsgeräten abtropft. Daher werden heute vielfach kapillare Auftragsgeräte verwendet, mit denen die erwähnten Handhabungsprobleme weitgehend gelöst sind. Problematisch bei kapillaren Auftragsgeräten ist, dass die Zubereitungen relativ niedrig viskos sein müssen, damit sie von einem Kapillarsystem eines Auftragsgeräts aus dessen Vorratsspeicher herausgefördert werden können. Sie müssen aber auch die an eine Zubereitung für den vorgesehenen Zweck gestellten Anforderungen erfüllen, d.h. sie sollen einen möglichst deckenden Farbauftrag ergeben, der eine für den Augenbereich ausreichende Permanenz aufweist und sich beim Abschminken leicht wieder entfernen lässt. Dies sind Eigenschaften, die mit niedrig viskosen Zubereitungen nur schwer zu erreichen sind, da diese wegen ihrer geringen Viskosität leicht in die Haut eindringen, was auch ihre Entfernung beim Abschminken erschwert. Niedrig viskose Zubereitungen neigen dazu in Fältchen und Hauttaschen einzudringen und darin zu verlaufen, so dass eingefärbte Hautareale mit einem einigermaßen glatten Rand nur schwer zu erzeugen sind. Es ergibt sich vielmehr ein eher ausgefranstes Erscheinungsbild. Was die technische Seite anbelangt, also die Verwendbarkeit einer Zubereitung für kapillare Auftragsgeräte, so reicht eine niedrige Viskosität alleine noch nicht aus. So muss etwa dafür Sorge getragen werden, dass die Zubereitung während des Nichtgebrauchs des Auftragsgeräts nicht eintrocknet und dadurch die Kapillarfunktion eines Auftragselements, etwa einer Sinterspitze, beeinträchtigt wird.

Davon ausgehend ist es die Aufgabe der Erfindung, eine kosmetische Zubereitung zum Färben der Augenlider und der Augenbrauen vorzuschlagen, die hinsichtlich der vorgenannten Nachteile verbessert ist, bei der insbesondere ein Verlaufen in Fältchen und Taschen der Haut vermieden ist.

Diese Aufgabe wird durch eine wässrige Zubereitung nach Anspruch 1 gelöst. Diese enthält 0,1 Gew.% bis 5 Gew.% Polyvinylpyrrolidon, 4 Gew.% bis 25 Gew. % eines Alkohols aus der Gruppe Ethanol, Propanol, 2 Gew.% bis 15 Gew.% eines Feuchthaltemittels, 0,1 Gew.% bis 5 Gew.% Polyoxyethylen-glycerin-fettsäureester und polyethermodifiziertes Polysiloxan, und ein Farbmittel, und weist eine Viskosität von weniger als 50 mPas (Brookfield, 25°C) auf.

Eine derartige Zubereitung lässt sich problemlos mit einem kapillaren Auftragsgerät auf ein Augenlid oder eine Augenbraue auftragen, wobei ein Verlaufen der Zubereitung in Fältchen und Hauttaschen vermieden ist, was vor allem dem Polyvinylpyrrolidon zu verdanken ist. Die Zubereitung trocknet auf der Haut leicht ab ohne dass sie ihr in einem störenden Ausmaß Feuchtigkeit entzieht. Andererseits zeigt die Zubereitung eine nur geringe Tendenz zum Austrocknen in dem kapillaren Auftragselement eines Auftragsgeräts, was insbesondere durch eine Kombination eines der oben aufgeführten Alkohole mit einem Feuchthaltemittel bewirkt wird. Der Alkohol verhindert ein Eintrocknen der Zubereitung während des Nichtgebrauchs des Auftragsgeräts. Eine eventuelle feuchtigkeitsentziehende Wirkung des Alkohols auf die Haut wird durch ein Feuchthaltemittel kompensiert, das auf der Haut die Funktion eines Feuchtigkeitsspenders hat. Um trotz der in der Zubereitung enthaltenen viskositätserhöhenden Substanzen, vor allem dem Vinylpyrrolidon und beispielsweise auch als Farbmittel zugesetzten Pigmenten für eine Anwendung in einem kapillaren Auftragsgerät ausreichende Viskositätswerte zu gewährleisten, ist Polyoxyethylen-glycerin-fettsäureester oder Polyether-modifiziertes Polysiloxan zugesetzt. Diese Substanzen wirken einer Viskositätserhöhung der Zubereitung entgegen, wobei eine Mischung dieser Substanzen besonders wirkungsvoll ist. Es war überraschend, dass die Zubereitung dennoch eine geringe Tendenz aufweist in Fältchen und Hauttaschen zu verlaufen, was in ganz besonderem Maße bei der Zugabe einer Mischung dieser Substanzen der Fall ist, wobei bevorzugt in Gehaltsgrenzen von 0,2 Gew.% bis 5 Gew.% - bezogen auf eine einzelne Substanz oder eine Subtanzmischung - gearbeitet wird.

Je nach den gewünschten Färbegraden beträgt der Anteil an Farbmitteln 0,1 Gew.% bis 15 Gew.%, wobei vorzugsweise Farbpigmente zum Einsatz kommen. Diese ergeben im Vergleich zu löslichen Farbstoffen in der Regel eine stärkere Färbung. Eine Anwendung in einem Kapillarsystem ist problemlos möglich, wenn eine Korngrößenverteilung der Pigmente von D90% < 10 µm eingehalten wird. Der Wassergehalt der Zubereitung reicht von 35 Gew.% bis 85 Gew.%.

Additive, etwa pflegende Mittel - wie Aloe Vera, Camilla Sinensis, Tocopherol oder Pantenol - und Konservierungsmitteln sind vorzugsweise auf insgesamt max. 5 Gew.% beschränkt.

### Beispiel 1 (nicht erfindungsgemäß):

Niederviskose Flüssigkeit zur Färbung von Augenbrauen

| | |
|---|---|
| Wasser dem. ⁴⁾ | 70,1 Gew% |
| Ethanol | 20,0 Gew% |
| Glycerin | 5,0 Gew% |
| PVP K 15 ¹⁾ | 2,0 Gew% |
| Polyether-modifiziertes Polysiloxan ²⁾ | 2,0 Gew% |
| Farbmittel | |
| C.I. 16035 (Rotfarbstoff) | 0,6 Gew% |
| C.I. 42090 (Blaufarbstoff) | 0,1 Gew% |
| C.I. 19140 (Gelbfarbstoff) | 0,2 Gew% |

Erhalten wird eine braune Flüssigkeit für Kapillarsysteme mit Pinselspitzen mit einer Viskosität von ca. 3,0 mPa s.

### Beispiel 2 (nicht erfindungsgemäß):

| | |
|---|---|
| Wasser dem. | 68,4 Gew% |
| 1,3-Butandiol | 12,0 Gew% |
| Ethanol | 8,0 Gew% |
| PVP K 15 ¹⁾ | 3,0 Gew% |
| Polyoxyethylen-glycerin-stearinsäure-ester ³⁾ (CAS 68553-11-7) | 0,5 Gew% |
| C.I. 42090 (Blaufarbstoff) | 7,8 Gew% |
| Camellia Sinensis Leaf Extract | 0,1 Gew% |
| Methylparaben | 0,2 Gew% |

Die erhaltene blaue Eyelinerlösung kann in kapillaren Auftragsgeräten mit flexibler Sinterspitze verwendet werden und weist eine Viskosität von ca. 4 mPa s (25°C Brookfield) auf.

### Beispiel 3:

Schwarze Flüssigkeit zur Färbung der Augenlider mit Schwarzpigment

| | |
|---|---|
| Wasser dem. | 65,0 Gew% |
| Polyoxyethylen-glycerin-stearinsäure-ester ³⁾ (CAS 68553-11-7) | 1,0 Gew% |
| Polyether-modifiziertes Polysiloxan ²⁾ | 0,5 Gew% |
| PVP K 30 ¹⁾ | 0,5 Gew% |
| Propylenglykol | 10,0 Gew% |
| Ethanol | 8,0 Gew% |
| C.I. 77266 (anorganisches Schwarzpigment) | 15,0 Gew% |

Schwarze Eyelinerflüssigkeit mit einer Viskosität von ca. 10 mPa s (Brookfield, 25°C).

Weitere Angaben zu den Beispielen:
1) z.B. unter der Bezeichnung PVP K 15 bzw. PVP K 30 von der Firma ISP, 50996 Köln erhältlich;
2) z.B. unter dem Handelnamen Abil B8851 von der Firma Goldschmidt, 45127 Essen erhältlich;
3) auch unter dem Namen PEG-30-Glycerylstearat bekannt; erhältlich z.B. unter dem Handelsnamen Tagat S von der Firma Goldschmidt, 45117 Essen;
4) dem. = demineralisiert

## Patentansprüche

1. Wässrige kosmetische Zubereitung zur Färbung der Augenlider und der Augenbrauen, die
- 0,1 Gew.% bis 5 Gew.% Polyvinylpyrrolidon,
- 4 Gew.% bis 25 Gew. % eines Alkohols aus der Gruppe Ethanol, Propanol,
- 2 Gew.% bis 15 Gew.% eines Feuchthaltemittels,
- 0,1 Gew.% bis 5 Gew.% Polyoxyethylen-glycerin-fettsäureester und polyethermodifiziertes Polysiloxan und ein Farbmittel enthält,
und die eine Viskosität von weniger als 50 mPas (Brookfield, 25°C) aufweist.

2. Zubereitung nach Anspruch 1,
**gekennzeichnet durch**
einen Anteil an Farbmittel von 0,1 Gew:% bis 15 Gew.%.

3. Zubereitung nach Anspruch 2,
**dadurch gekennzeichnet,**
**dass** als Farbmittel Pigmente mit einer Korngrößenverteilung D90% < 10 µm enthalten sind.

4. Zubereitung nach einem der vorhergehenden Ansprüche,
**dadurch gekennzeichnet,**
**dass** max. 5 Gew.% Additive enthalten sind.

5. Zubereitung nach einem der vorhergehenden Ansprüche,
**dadurch gekennzeichnet,**
**dass** sie 35 Gew.% bis 85 Gew.% Wasser enthält.

## Claims

1. Aqueous cosmetic preparation for colouring eyelids and eyebrows, comprising
- 0.1% by weight to 5% by weight of polyvinylpyrrolidone,
- 4% by weight to 25% by weight of an alcohol selected from the group consisting of ethanol and propanol,
- 2% by weight to 15% by weight of a dampening agent,
- 0.1% by weight to 5% by weight of polyoxyethylene glycerol fatty acid ester and polyether-modified polysiloxane and a colorant
and having a viscosity of less than 50 mPas (Brookfield, 25°C).

2. Preparation according to Claim 1, **characterized by** a colorant proportion of 0.1% by weight to 15% by weight.

3. Preparation according to Claim 2; **characterized in that** pigments having a particle size distribution D90% < 10 µm are present as colorant.

4. Preparation according to any preceding claim, **characterized in that** a maximum of 5% by weight of additives is included.

5. Preparation according to any preceding claim, **characterized in that** it contains 35% by weight to 85% by weight of water.

## Revendications

1. Préparation cosmétique aqueuse pour colorer les paupières et les sourcils, qui contient
- de 0,1 % en poids à 5 % en poids de polyvinyl-pyrrolidone,
- de 4 % en poids à 25 % en poids d'un alcool choisi dans le groupe éthanol, propanol,
- de 2 % en poids à 15 % en poids d'un agent conservant l'humidité,
- de 0,1 % en poids à 5 % en poids de polyoxyéthylène-glycérine-esther d'acide gras et de polysiloxane modifié par un polyéther et un colorant,
et qui a une viscosité de moins de 50 mPas (Brookfield, 25°C).

2. Préparation suivant la revendication 1,
**caractérisée par**
une proportion de colorant de 0,1 % en poids à 15 % en poids.

3. Préparation suivant la revendication 2,
**caractérisée en ce qu'**
elle contient comme colorant des pigments ayant une répartition granulométrique D90 % < 10 µm.

4. Préparation suivant l'une des revendications précédentes,
**caractérisée en ce qu'**
elle contient au maximum 5 % en poids d'additifs.

5. Préparation suivant l'une des revendications précédentes,
**caractérisée en ce qu'**
elle contient de 35 % en poids à 85 % en poids d'eau.
